# EUROPEAN PATENT APPLICATION

(11) **EP 4 635 430 A1**
(43) Date of publication of application: **22.10.2025**
(21) Application number: 23903507.4
(22) Date of filing: 12.12.2023
(51) Int. Cl.: A61B 17/29

(54) **JAW ASSEMBLY AND SURGICAL INSTRUMENT USING SAME**

(30) Priority: 13.12.2022 JP 2022198865
(71) Applicant: NHK Spring Co., Ltd., Yokohama-shi, Kanagawa 236-0004 (JP)
(72) Inventor: ISHIYAMA Yuta, Yokohama-shi, Kanagawa 236-0004 (JP); HIRATA Takafumi, Yokohama-shi, Kanagawa 236-0004 (JP); HAYAKAWA Yuki, Yokohama-shi, Kanagawa 236-0004 (JP)
(74) Representative: Becker Kurig & Partner Patentanwälte mbB
(86) International application number: PCT/JP2023/044430
(87) International publication number: WO 2024/128223

(57) **Abstract**

Provided is a jaw assembly that has an increased jaw-closing force. The jaw assembly comprises: a pair of jaws 15 that are opened and closed between each other; a support portion 13 that rotatably supports the pair of jaws 15 to enable opening and closing thereof; and a movable member 17 that is relatively rotatably coupled with the pair of jaws 15 and can be reciprocally moved relative to the support portion 13. The support portion 13 includes a recess portion 25 opening on either side in an intersecting direction with respect to a reciprocal movement direction of the movable member 17. The pair of jaws 15 each include a rotational axis portion 31 that engages the recess portion 25 axially rotatably from the outside in the intersecting direction.

## Description

### Technical Field

The present invention relates to a jaw assembly supplied to robots, manipulators, etc., and a surgical instrument using the same.

### Related Art

As a conventional jaw assembly, there is one as disclosed in Patent Literature 1, for example, in which a pair of jaws that are opened and closed between each other are rotatably supported on a sleeve by a pin.

In this jaw assembly, an operating wire that can reciprocally move is coupled to each of the pair of jaws, and the pair of jaws are rotated around the pin by the reciprocal movement of the operating wire to enable opening and closing.

In addition, it is possible to position the coupling part between the jaw and the operating wire, which serves as the point of force application, away from the pin, which serves as the fulcrum and rotation center, making it possible to increase the force acting between the jaws in response to the operation of the operating wire.

However, in such a jaw assembly, there is a limitation to the position where the pin could be placed, and there is also a limitation to the improvement of the force acting between the jaws.

### Citation List

### Patent Literature

Patent Literature 1: Japanese translation of PCT international application No. 2020-508825.

### SUMMARY OF INVENTION

### Technical Problem

The problem to be solved is that there is a limitation to the improvement of the force acting between the jaws.

### Solution to Problem

The present invention provides a jaw assembly, including: a pair of jaws that are opened and closed between each other; a support portion that rotatably supports at least one of the pair of jaws and enables opening and closing; and a movable member that is relatively rotatably coupled to at least one of the pair of jaws that is rotatably supported by the support portion, and that is capable of reciprocal movement relative to the support portion. The support portion includes a recess portion that opens at an edge portion in an intersecting direction with respect to a reciprocal movement direction of the movable member. At least one of the pair of jaws that is rotatably supported by the support portion includes a rotational axis portion that engages with the recess portion axially rotatably from outside in the intersecting direction.

The present invention also provides a surgical instrument using the jaw assembly.

### Effects of Invention

According to the present invention, the force acting between the jaws may be improved.

### BRIEF DESCRIPTION OF DRAWINGS

[FIG. 1] FIG. 1 is a front view showing pliers as a surgical instrument using a jaw assembly according to Embodiment 1 of the present invention.
[FIG. 2] FIG. 2 is a perspective view showing a closed state of the jaw assembly of the pliers of FIG. 1.
[FIG. 3] FIG. 3 is a perspective view showing an open state of the jaw assembly of FIG. 2.
[FIG. 4] FIG. 4 is an exploded perspective view of the jaw assembly of FIG. 2.
[FIG. 5] FIG. 5 is a front view showing the relationship between the rotation center of the jaw and the protrusion in the jaw assembly of FIG. 2.
[FIG. 6] FIG. 6(A) to FIG. 6(C) are perspective views showing the opening and closing of the jaw assembly of FIG. 2.
[FIG. 7] FIG. 7(A) to FIG. 7(C) are front views showing the opening and closing of the jaw assembly of FIG. 2.
[FIG. 8] FIG. 8 is a perspective view showing a part of the jaw assembly according to Embodiment 2 of the present invention.
[FIG. 9] FIG. 9(A) and FIG. 9(B) are perspective views showing the jaw of the jaw assembly of FIG. 8 from the surface side and the back surface side, respectively.
[FIG. 10] FIG. 10(A) to FIG. 10(C) show the closed state of the jaw assembly according to Embodiment 3 of the present invention, where FIG. 10(A) is a perspective view, FIG. 10(B) is a front view, and FIG. 10(C) is a side view.
[FIG. 11] FIG. 11(A) to FIG. 11(C) show the open state of the jaw assembly of FIG. 10(A) to FIG. 10(C), where FIG. 11(A) is a perspective view, FIG. 11(B) is a front view, and FIG. 11(C) is a side view.

### DESCRIPTION OF EMBODIMENTS

The objective of improving the force acting between the jaws is achieved by the following configuration.

As shown in the figure, the jaw assembly 7 includes a pair of jaws 15, a support portion 13, and a movable member 17.

The pair of jaws 15 are configured to open and close between each other. The support portion 13 rotatably supports at least one of the pair of jaws 15 to enable the opening and closing of the pair of jaws 15. The movable member 17 is a member that is relatively rotatably coupled to at least one of the pair of jaws 15 that is rotatably supported by the support portion 13, and that is capable of reciprocal movement relative to the support portion 13.

In the case of single-sided opening, only one of the pair of jaws 15 is rotatably coupled to the support portion 13, and in the case of double-sided opening, both jaws are rotatably coupled to the support portion 13.

The support portion 13 includes a recess portion 25 that opens at an edge portion in an intersecting direction with respect to a reciprocal movement direction of the movable member 17, and at least one of the pair of jaws 15 that is rotatably supported by the support portion 13 includes a rotational axis portion 31 that engages with the recess portion 25 axially rotatably from outside in the intersecting direction of the recess portion 25.

The jaw assembly 7 may be configured as a cam type, link type, etc. In the case of a cam type, a cam portion 19 is provided. The cam portion 19 is provided between the support portion 13 and at least one of the pair of jaws 15 rotatably supported by the support portion 13, and rotates at least one of the pair of jaws 15 rotatably supported by the support portion 13 to perform the opening and closing of the pair of jaws 15 in response to the reciprocal movement of the movable member 17. This cam portion 19 may maintain the engagement of the rotational axis portion 31 with the recess portion 25.

At least one of the jaws 15 rotatably supported by the support portion 13 may have a rotation center RC and a coupling part to the movable member 17 positioned apart from each other in an intersecting direction with respect to the reciprocal movement direction of the movable member 17, at least in the closed state of the pair of jaws 15. In this case, the rotation center RC and the coupling part of each jaw 15 may be positioned on opposite sides in the intersecting direction across the opening and closing center OC of the pair of jaws 15.

The movable member 17 may include one of either a pair of opening portions 17a or protrusions 33 as the coupling part, and the pair of jaws 15 may each include the other of the opening portions 17a or protrusions 33 that engage with the one of the pair of opening portions 17a or protrusions 33.

The opening portion 17a is an elongated hole that allows relative movement of the protrusion 33 in the intersecting direction, and before maintaining the engagement of the rotational axis portion 31 with the recess portion 25, engagement and disengagement of the rotational axis portion 31 with respect to the recess portion 25 may be permitted.

The jaw assembly 7 is applicable to various robots and manipulators, and may be used, for example, in surgical instruments.

### Embodiment 1

### [Pliers]

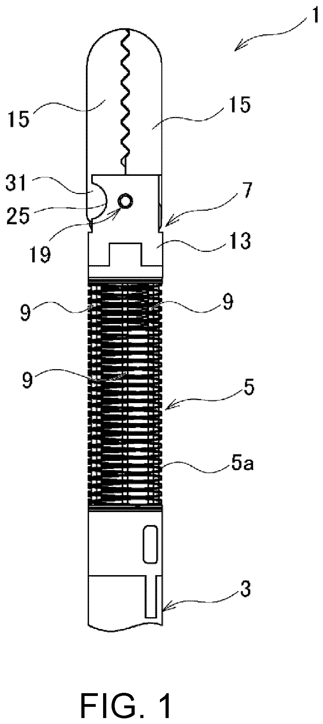

FIG. 1 is a front view showing pliers as a surgical instrument using a jaw assembly according to Embodiment 1 of the present invention.

The pliers 1 of this embodiment is a surgical instrument used in surgical assistance robots or surgical assistance manipulators, and includes a shaft 3, a bending portion 5, and a jaw assembly 7.

In this example, the pliers 1 is shown as an example of a surgical instrument, but as long as it performs opening and closing of the jaws 15 to be described later, it may be a surgical instrument having various functions such as grasping, separation, cutting, etc. Further, the jaw assembly 7 may be applied to devices other than surgical instruments, such as robots or manipulators having various functions including grasping, separation, cutting, etc.

The shaft 3 is formed in a cylindrical shape and is axially rotatably coupled, for example, to a surgical assistance robot or surgical assistance manipulator. The bending portion 5 is provided at the tip end of the shaft 3. It is noted that the shape of the shaft 3 is not particularly limited.

The bending portion 5 has a joint function and is a part that enables flexion extension of the pliers 1. The bending portion 5 of this embodiment is formed by laminating multiple wave washers 5a in the axial direction and maintaining the laminated state by welding or the like. It is noted that the bending portion 5 is not particularly limited as long as it enables flexion and extension, and it may also be a coil spring or bellows. The axial direction refers to the direction along the axis of the pliers 1.

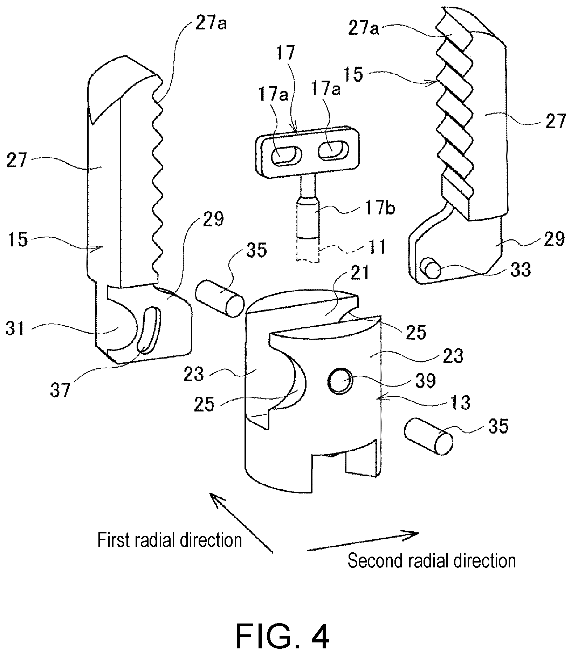

In such a bending portion 5, wires 9 are inserted through in the axial direction at predetermined intervals in the circumferential direction, and flexion extension is performed by operating the wires 9. The circumferential direction refers to the direction along the outer circumference of the pliers 1. Inside the bending portion 5, a core material (not shown) for suppressing compression in the axial direction and an operation member 11 (see FIG. 4) for opening and closing the jaws 15 to be described later are provided.

The operation member 11 is a push-pull cable and causes the movable member 17 to be described later to perform reciprocal movement in the axial direction. It is noted that the operation member 11 may also be an air tube or the like as long as it causes the movable member 17 to perform reciprocal movement.

The jaw assembly 7 is supported at the tip end of the bending portion 5.

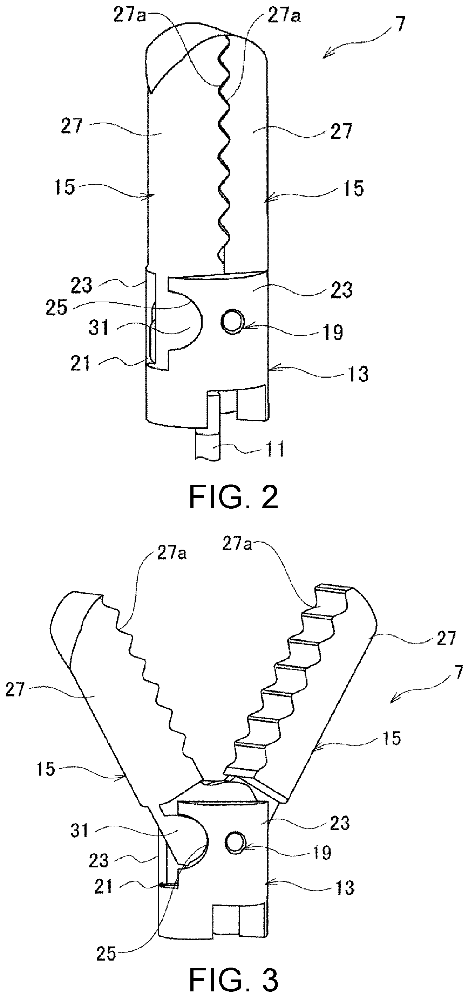

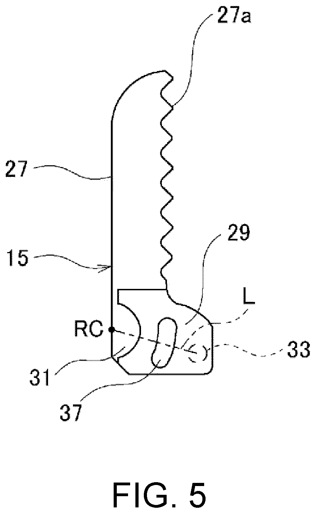

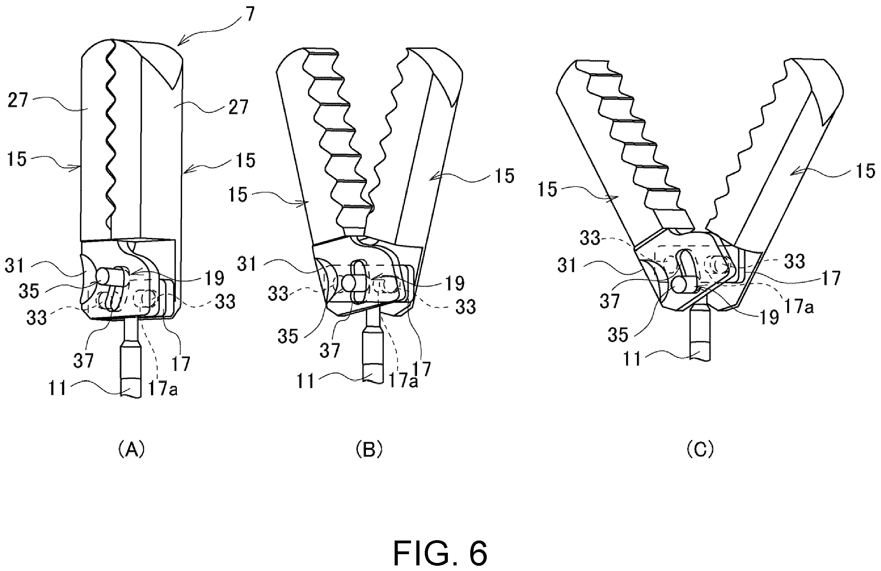

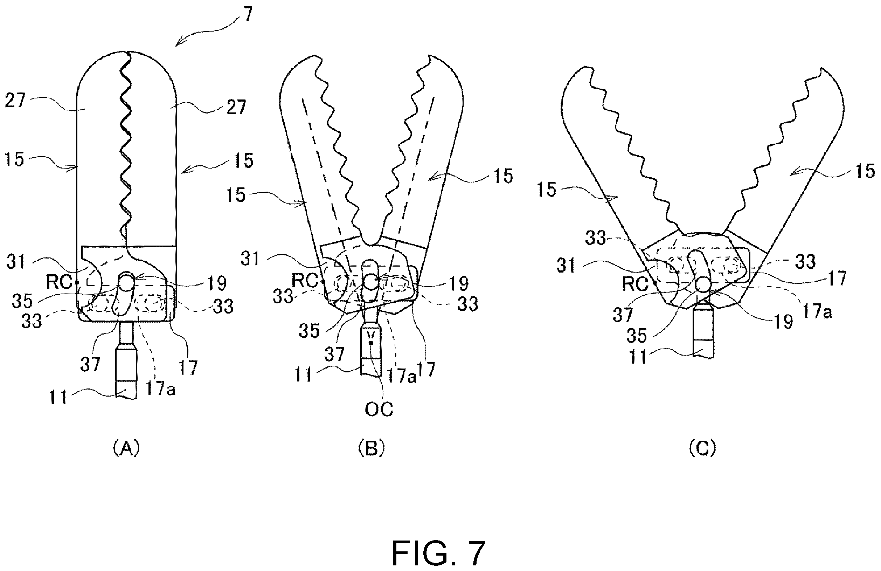

FIG. 2 is a perspective view showing a closed state of the jaw assembly of the pliers of FIG. 1, and FIG. 3 is a perspective view showing an open state of the same. FIG. 4 is an exploded perspective view of the jaw assembly of FIG. 2. FIG. 5 is a front view showing the relationship between the rotation center of the jaw and the protrusion in the jaw assembly of FIG. 2. FIG. 6(A) to FIG. 6(C) are perspective views showing the opening and closing of the jaw assembly of FIG. 2, and FIG. 7(A) to FIG. 7(C) are front views of the same. It is noted that in FIG. 6(A) to FIG. 7(C), the support portion 13 is omitted.

The jaw assembly 7 includes a support portion 13, a pair of jaws 15, a movable member 17, and a cam portion 19.

The support portion 13 is coupled to the bending portion 5, and as shown in FIG. 2 to FIG. 4, rotatably supports the pair of jaws 15 to enable opening and closing. In this embodiment, the pair of jaws 15 are both supported by the support portion 13 as a double-sided opening type. The support portion 13 is formed in a generally cylindrical shape, but is not particularly limited. This support portion 13 has a slit 21 provided on the tip end side in the axial direction, and support wall portions 23 that support the jaws 15 are partitioned on one side and the other side in the radial direction across the slit 21 (hereinafter, first radial direction).

This support portion 13 has recess portions 25 that open on both sides in the radial direction along the slit 21 (hereinafter, second radial direction). It is noted that the second radial direction is perpendicular to the first radial direction in plan view. The radial direction refers to a direction along the diameter of the pliers 1, and becomes an intersecting direction with respect to the reciprocal movement direction of the movable member 17.

The recess portions 25 are provided in each support wall portion 23, and in a front view as viewed from the first radial direction, they are formed in oppositely facing fan shapes, particularly semicircular shapes. These recess portions 25 open outward with the chord of the fan shape (diameter of the semicircle) positioned at the side edge of the support wall portion 23 in the second radial direction.

The inner surface of the recess portion 25 gradually becomes larger in the first radial direction from the side edge of the support wall portion 23 toward the inside in the second radial direction in accordance with the outer circumferential shape of the support portion 13. It is noted that the shape of the recess portion 25 is arbitrary, as long as it may axially rotatably support the rotational axis portion 31 described later.

The pair of jaws 15, as shown in FIG. 2 to FIG. 7(C), are configured to open and close between each other, and in this embodiment, are constructed as grippers that perform holding through a closing operation. These jaws 15 are formed in the same shape and are arranged with their front and back sides reversed in the first radial direction. However, the jaws 15 do not need to be of the same shape, and may have different shapes. Each jaw 15 includes a main body portion 27, an arm 29, and a rotational axis portion 31.

The main body portion 27 is formed in a rod shape and is the part that performs the opening and closing operation. The main body portion 27 has a wave-shaped meshing portion 27a that meshes with each other in a closed state in the second radial direction. At the base end of the main body portion 27 in the axial direction, the rotational axis portion 31 is provided via the arm 29.

The arm 29 is formed in a plate shape and has a smaller thickness in the first radial direction than the main body portion 27. This arm 29 protrudes in the axial direction from the base end of the main body portion 27 and also protrudes in the second radial direction, which is the intersecting direction, from the meshing portion 27a. This arm 29 is inserted into the slit 21 of the support portion 13.

On one side of the arm 29 in the second radial direction, the rotational axis portion 31 is provided, and on the other side of the same, a protrusion 33 is provided. Further, between the rotational axis portion 31 and the protrusion 33, a cam groove 37 of the cam portion 19 to be described later is provided.

The rotational axis portion 31, in a front view as viewed from the first radial direction, is formed in a fan shape, particularly a semicircular shape. This semicircular rotational axis portion 31 engages with the semicircular recess portion 25 of the support portion 13 from the outside in the second radial direction. As a result, the rotational axis portion 31 is axially rotatably supported by sliding in the recess portion 25. It is noted that the configuration of the rotational axis portion 31 may be in other shapes such as rod-shaped, as long as it is axially rotatably supported by sliding in the recess portion 25. This engagement state of the rotational axis portion 31 of the jaw 15 with the recess portion 25 of the support portion 13 is maintained by the cam portion 19 as described later.

This rotational axis portion 31, similar to the recess portion 25, gradually increases in dimension in the first radial direction from both side edges of the support wall portion 23 toward the inside in the second radial direction. In this embodiment, the semicircular shapes of the rotational axis portion 31 and the recess portion 25 have matching radii of curvature and depths in the second radial direction. As a result, the rotation center RC is positioned at the side edge of the support portion 13 in the second radial direction.

It is possible to position the rotation center RC outside the support portion 13 in the second radial direction by making the recess portion 25 shallower in the second radial direction in relation to the rotational axis portion 31. In this embodiment, the recess portion 25 may be made shallower by making the central angle of the recess portion 25 smaller than the central angle of the rotational axis portion 31.

Conversely, it is possible to position the rotation center RC outside the support portion 13 in the second radial direction by making the recess portion 25 deeper in the second radial direction in relation to the rotational axis portion 31. In this embodiment, the recess portion 25 may be made deeper by making the central angle of the recess portion 25 larger than the central angle of the rotational axis portion 31.

Thus, the position of the rotation center RC may be easily set by adjusting the recess portion 25 and the rotational axis portion 31.

The protrusion 33 serves as a coupling part for the movable member 17 to be described later. In this embodiment, the protrusion 33 is provided protruding from the surface on the opposite side of the arm 29 with respect to the rotational axis portion 31 in the first radial direction. The shape of the protrusion 33 is cylindrical, but is not particularly limited.

This protrusion 33, in the closed state of the pair of jaws 15, is positioned apart from the rotation center RC in the second radial direction. In this embodiment, it is positioned on the opposite side of the rotation center RC across the opening and closing center OC (FIG. 7(B)) of the main body portion 27 of the jaw 15. The opening and closing center OC is the central position for setting the opening and closing angle of the main body portion 27 of the jaw 15, and in this embodiment, it is positioned at the center of the support portion 13 in the second radial direction.

The jaws 15 are opened and closed by the movable member 17 through this protrusion 33.

The movable member 17 is a member that is relatively rotatably coupled to both of the pair of jaws 15, and that is capable of reciprocal movement relative to the support portion 13. The movable member 17 is formed in a plate shape and is positioned between the arms 29 of the jaws 15 in the first radial direction.

The movable member 17 is provided with a pair of opening portions 17a that penetrate in the first radial direction. These opening portions 17a are arranged in parallel in the second radial direction, and the protrusions 33 of the jaws 15 are inserted in the first radial direction. It is noted that the protrusions 33 may be provided on the movable member 17, and the opening portions 17a may be provided on the jaws 15. Thus, one of the protrusions 33 and the opening portions 17a may be provided on the movable member 17, and the other may be provided on the jaws 15.

In this embodiment, the opening portion 17a is formed as an elongated hole that is long in the second radial direction. This allows the opening portion 17a to enable relative movement of the protrusion 33 in the second radial direction, and before maintaining the engagement of the rotational axis portion 31 with the recess portion 25, engagement and disengagement of the rotational axis portion 31 with respect to the recess portion 25 are permitted. Thus, when engaging the rotational axis portion 31 with the recess portion 25, the protrusion 33 may be inserted into the opening portion 17a in advance, and by moving the protrusion 33 in the second radial direction within the opening portion 17a, the operation may be performed easily.

The movable member 17 is integrally provided with a tubular connection portion 17b that is connected to the operation member 11. With this connection portion 17b, the movable member 17 is connected to the operation member 11, enabling reciprocal movement by the operation member 11.

Through this reciprocal movement, the movable member 17 applies force around the rotation center RC to the jaws 15 via the protrusion 33, which serves as the coupling part. At this time, since the rotation center RC has a shape where the rotational axis portion 31 of the jaw 15 engages with the recess portion 25 of the support portion 13 from the outside in the second radial direction, the distance L between the protrusion 33 and the rotation center RC may be increased to improve the rotational force of the jaw 15. As a result, it becomes possible to enhance the force acting between the pair of jaws 15 in response to the operation.

In this embodiment, by positioning the rotation center RC at the side edge of the support portion 13 in the second radial direction, it becomes possible to position the rotation center RC as far outside as possible while suppressing the enlargement of the pliers 1 and the jaw assembly 7 in the second radial direction.

The cam portions 19 are provided between the support portion 13 and each of the pair of jaws 15, and rotate the pair of jaws 15 to open and close in response to the reciprocal movement of the movable member 17.

The cam portion 19 may adopt various forms, but in this embodiment, it consists of a cam pin 35 attached to the support portion 13 and cam grooves 37 provided on each jaw 15.

The cam pin 35 is attached to a through hole 39 of the support portion 13, with its tip end protruding into the slit 21 of the support portion 13 in the first radial direction. Inside the slit 21, the tip end of the cam pin 35 engages with the cam groove 37. The cam pin 35 has a cylindrical shape, but is not particularly limited to this shape.

The cam groove 37 is provided on the arm 29 of each jaw 15. The cam groove 37 is formed in an arc shape around the rotation center RC. It is noted that the cam groove 37 consists of a recess portion, but may also be a through hole. By the cam pin 35 relatively moving along this cam groove 37, the opening and closing operation of the jaws 15 is performed.

Further, since the cam groove 37 and the cam pin 35 of the cam portion 19 restrict the movement of the jaw 15, it becomes possible to maintain the engagement of the rotational axis portion 31 with the recess portion 25. Thus, it becomes unnecessary to separately maintain the engagement of the rotational axis portion 31 with the recess portion 25, enabling simplification of the structure.

In addition, in the case where the protrusion 33 and the rotation center RC are separated in the second radial direction as in this embodiment, it is also possible to omit the cam portion 19. In this case, the engagement state between the rotational axis portion 31 of the jaw 15 and the recess portion 25 of the support portion 13 may be maintained by the movable member 17.

### [Operation]

The pliers 1 of this embodiment may open and close the jaws 15 by causing the movable member 17 to perform reciprocal movement in the axial direction through the operation member 11, as shown in FIG. 6(A) to FIG. 7(C).

Specifically, as shown in FIG. 6(A) and FIG. 7(A), in the closed state of the pair of jaws 15, the movable member 17 is in a retracted position located on the bending portion 5 side in the axial direction. When this movable member 17 in the retracted position is advanced in the axial direction to the opposite side with respect to the bending portion 5, as shown in FIG. 6(B) and FIG. 6(C) as well as FIG. 7(B) and FIG. 7(C), the protrusion 33, which is the coupling part, advances in the axial direction together with the movable member 17.

At this time, since the rotation center RC and the protrusion 33 are positioned separated in the second radial direction, the pair of jaws 15 receive a force around the rotation center RC in the opening direction. Furthermore, the pair of jaws 15 receive a more reliable force around the rotation center RC in the opening direction due to the function of the cam portion 19. As a result, the pair of jaws 15 rotate with respect to the rotation center RC, and the space between the main body portions 27 opens.

In this embodiment, since the rotational axis portion 31 of the jaw 15 has a shape that engages with the recess portion 25 of the support portion 13 from the outside in the second radial direction, it is possible to increase the distance between the protrusion 33 and the rotation center RC to improve the rotational force of the jaw 15. As a result, it becomes possible to improve the opening force acting between the pair of jaws 15 in relation to the operational force of the movable member 17.

Similarly, as shown in FIG. 6(B) and FIG. 6(C) as well as FIG. 7(B) and FIG. 7(C), in the open state of the jaws 15, when the movable member 17, which is in an advanced position biased to the opposite side with respect to the bending portion 5 in the axial direction, is retracted toward the bending portion 5 side, as shown in FIG. 6(A) and FIG. 6(B) as well as FIG. 7(A) and FIG. 7(B), the pair of jaws 15 rotate with respect to the rotation center RC, and the space between the main body portions 27 closes. At this time, since it is possible to improve the rotational force of the jaw 15, it becomes possible to improve the closing force acting between the pair of jaws 15 in relation to the operational force of the movable member 17.

Thus, with the pliers 1, it becomes possible to reliably perform holding with the jaws 15 using less operational force.

### Embodiment 2

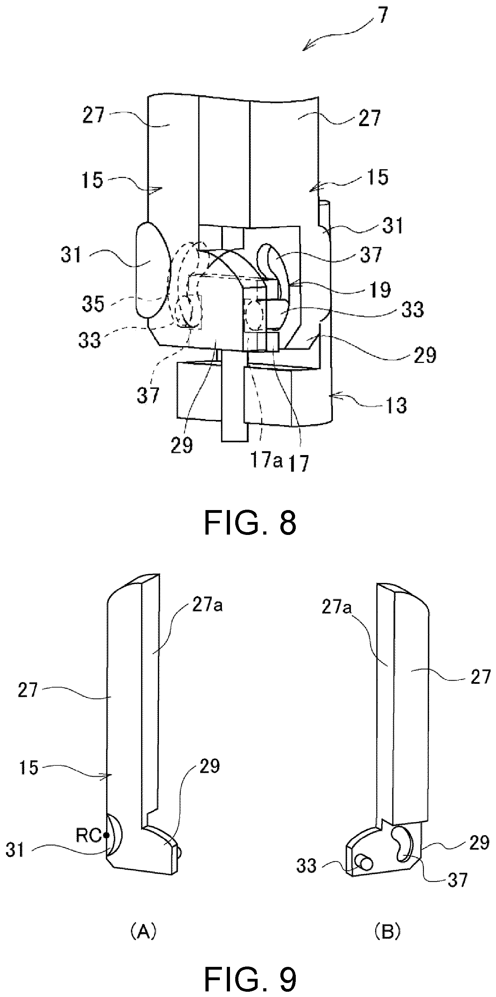

FIG. 8 is a perspective view showing a part of the jaw assembly according to Embodiment 2 of the present invention. FIG. 9(A) and FIG. 9(B) are perspective views showing the jaw of the jaw assembly of FIG. 8 from the surface side and the back surface side, respectively. In Embodiment 2, the same reference numerals are used for configurations corresponding to those in Embodiment 1, and redundant description will be omitted.

In this embodiment, the cam pin 35 of the cam portion 19 is omitted, and the protrusion 33 serves the role of the cam pin 35.

That is, the arm 29 of the jaw 15 has the rotational axis portion 31 provided on one side in the second radial direction, and on the other side of the same, the protrusion 33 is provided on the surface opposite to the surface where the rotational axis portion 31 is provided in the first radial direction. Further, the arm 29 has the cam groove 37 of the cam portion 19 provided on one side in the second radial direction on the same surface as the protrusion 33.

The cam groove 37 is formed in an arc shape around the rotation center RC of the counterpart jaw 15. The tip end of the protrusion 33 of the counterpart jaw 15 engages with this cam groove 37. The protrusion 33 protrudes from the arm 29 in the first radial direction and engages with the opening portion 17a of the movable member 17 at its middle portion. The opening portion 17a is concave with the outer side in the second radial direction being released. The tip end of this protrusion 33 protrudes from the movable member 17 and engages with the cam groove 37 of the counterpart jaw 15. Other aspects are the same as in Embodiment 1.

In Embodiment 2 having such a configuration, the same effects as in Embodiment 1 may be achieved.

### Embodiment 3

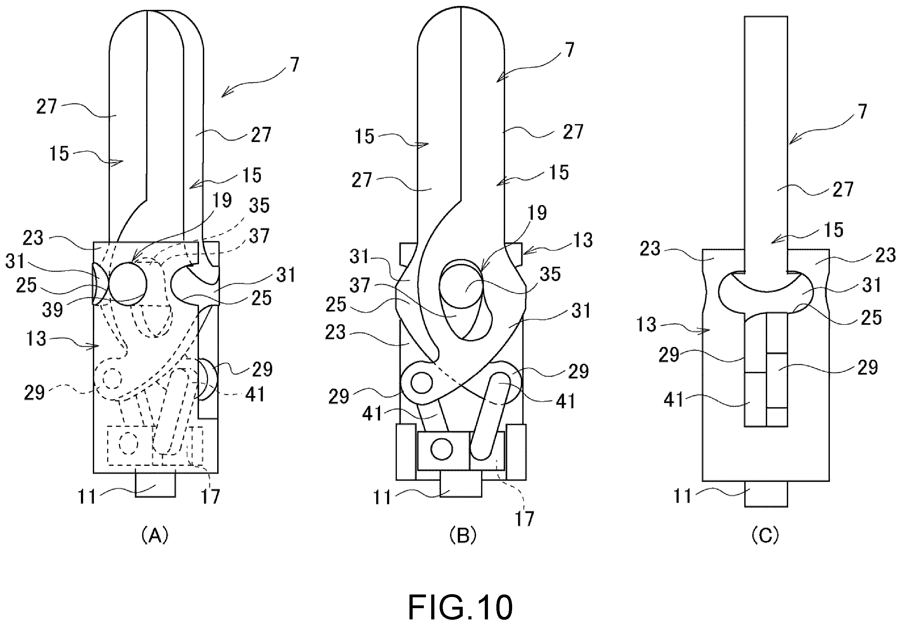

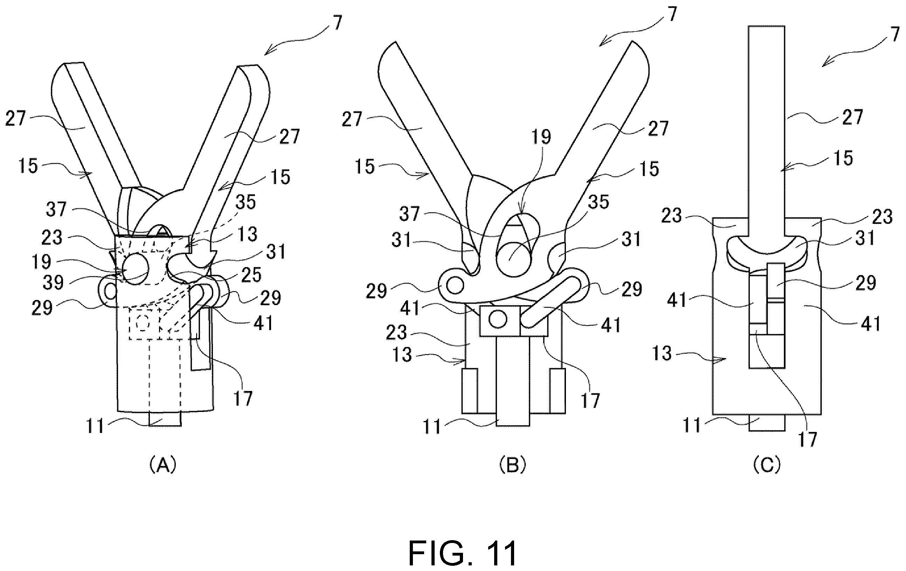

FIG. 10(A) to FIG. 10(C) show the closed state of the jaw assembly according to Embodiment 3 of the present invention, where FIG. 10(A) is a perspective view, FIG. 10(B) is a front view, and FIG. 10(C) is a side view. FIG. 11(A) to FIG. 11(C) show the open state of the jaw assembly of FIG. 10(A) to FIG. 10(C), where FIG. 11(A) is a perspective view, FIG. 11(B) is a front view, and FIG. 11(C) is a side view. It is noted that in FIG. 10(B) and FIG. 11(B), a part of the support portion 13 is omitted to expose the internal structure. Further, in Embodiment 3, the same reference numerals are used for configurations corresponding to those in Embodiment 1, and redundant description will be omitted.

In this embodiment, the jaw assembly 7 is configured as a link type. That is, the coupling part of each jaw 15 to the movable member 17 is constructed with a link 41.

The link 41 is rod-shaped in this embodiment. One side in the longitudinal direction of the link 41 is rotatably coupled to the other side in the second radial direction of the jaw 15, and the other side in the longitudinal direction of the link 41 is rotatably coupled to the movable member 17. It is noted that the link 41 does not need to be rod-shaped as long as it is positioned across the jaw 15 and the movable member 17 and may be coupled to them.

In response to the reciprocal movement of the movable member 17 in the axial direction, such link 41 rotates around the other side in the longitudinal direction that is coupled to the movable member 17. Through this rotation, the jaw 15 rotates around the rotational axis portion 31 to open and close.

Further, in this embodiment, the cam portion 19 has a cam pin 35 that is shared between the pair of jaws 15. That is, the cam groove 37 is provided to penetrate through the arm 29 of the jaw 15 in the first radial direction. The cam grooves 37 of the pair of jaws 15 partially overlap in the first radial direction. A common cam pin 35 is inserted through the cam grooves 37 of this pair of jaws 15. Both ends of the cam pin 35 are engaged with the through holes 39 provided in the support portion 13.

Furthermore, in this embodiment, the rotational axis portion 31 of each jaw 15 is provided to protrude on both sides in the first radial direction relative to the main body portion 27. The support wall portions 23 of the support portion 13 each include recess portions 25 on both sides in the second radial direction. The rotational axis portion 31 is rotatably engaged with the recess portions 25 on both sides in the first radial direction. As a result, the rotational axis portion 31 is supported to bridge across both support wall portions 23 of the support portion 13 in the first radial direction. Other configurations are the same as in Embodiment 1.

In Embodiment 3 having such a configuration, the same effects as in Embodiment 1 may be achieved. Further, in this embodiment, since both sides of the rotational axis portion 31 in the first radial direction are rotatably engaged with the recess portions 25, the support and operation of the rotational axis portion 31 may be more easily stabilized.

### Reference Signs List

1 Pliers
7 Jaw assembly
13 Support portion
15 Jaw
17 Movable member
17a Opening portion
19 Cam portion
25 Recess portion
31 Rotational axis portion
33 Protrusion
35 Cam pin
37 Cam groove
RC Rotation center
OC Opening and closing center

## Claims

1. A jaw assembly, comprising:
a pair of jaws that are opened and closed between each other;
a support portion that rotatably supports at least one of the pair of jaws and enables opening and closing; and
a movable member that is relatively rotatably coupled to at least one of the pair of jaws that is rotatably supported by the support portion, and that is capable of reciprocal movement relative to the support portion,
wherein the support portion comprises a recess portion that opens at an edge portion in an intersecting direction with respect to a reciprocal movement direction of the movable member, and
at least one of the pair of jaws that is rotatably supported by the support portion comprises a rotational axis portion that engages with the recess portion axially rotatably from outside in the intersecting direction.

2. The jaw assembly according to claim 1, comprising:
a cam portion provided between the support portion and at least one of the pair of jaws that is rotatably supported by the support portion, and the cam portion being configured to rotate at least one of the pair of jaws that is rotatably supported by the support portion to perform opening and closing in response to a reciprocal movement of the movable member,
wherein the cam portion maintains an engagement state of the rotational axis portion with respect to the recess portion.

3. The jaw assembly according to claim 1 or 2, wherein
at least one of the pair of jaws that is rotatably supported by the support portion has a rotation center and a coupling part to the movable member positioned apart from each other in an intersecting direction with respect to a reciprocal movement direction of the movable member, at least in a closed state of the pair of jaws.

4. The jaw assembly according to claim 3, wherein
the rotation center and the coupling part are positioned on opposite sides in the intersecting direction across an opening and closing center of the pair of jaws, at least in a closed state of the pair of jaws.

5. The jaw assembly according to claim 1 or 2, wherein
the movable member comprises one of an opening portion and a protrusion, and
at least one of the pair of jaws rotatably supported by the support portion comprises the other of the opening portion and the protrusion that engages with the one of the opening portion and the protrusion.

6. The jaw assembly according to claim 5, wherein
the opening portion is an elongated hole that allows relative movement of the protrusion in the intersecting direction, and
before maintaining engagement of the rotational axis portion with the recess portion, engagement and disengagement of the rotational axis portion with respect to the recess portion are permitted.

7. A surgical instrument using the jaw assembly according to claim 1.
